# FASCICULE DE BREVET EUROPEEN

(11) **EP 1 485 134 B1**
(45) Date de publication et mention de la délivrance du brevet: **21.06.2006**
(21) Numéro de dépôt: 03718874.5
(22) Date de dépôt: 14.02.2003
(51) Int. Cl.: A61L 2/24, A61L 2/18

(54) **METHODE ET DISPOSITIF DE MESURE ET DE CONTROLE DE LA CIRCULATION DES FLUIDES DANS LES CANAUX DES ENDOSCOPES**
VERFAHREN UND VORRICHTUNG ZUR MESSUNG UND KONTROLLE DES KREISLAUFS VON FLÜSSIGKEITEN IN ENDOSKOPKANÄLEN
METHOD AND DEVICE FOR MEASURING AND CONTROLLING THE CIRCULATION OF FLUIDS IN ENDOSCOPE CHANNELS

(30) Priorité: 20.03.2002 FR 0203439
(43) Date de publication de la demande: 15.12.2004
(73) Titulaire: Mariotti, Bernard, 13007 Marseille (FR); Dray, Frédéric, 13010 Marseille (FR)
(72) Inventeur: Mariotti, Bernard, 13007 Marseille (FR); Dray, Frédéric, 13010 Marseille (FR)
(74) Mandataire: Roman, Michel
(86) Numéro de dépôt international: PCT/FR2003/000479
(87) Numéro de publication internationale: WO 2003/077960

(56) Documents cités:
- EP-A- 0 945 140
- WO-A-93/24046
- FR-A- 2 705 896
- FR-A- 2 803 755
- PATENT ABSTRACTS OF JAPAN vol. 2002, no. 07, 3 juillet 2002 (2002-07-03) & JP 2002 065607 A (OLYMPUS OPTICAL CO LTD), 5 mars 2002 (2002-03-05)

## Description

La présente invention concerne une méthode et un dispositif de mesure et de contrôle de la circulation des fluides dans les canaux des endoscopes ayant pour objet la vérification et l'enregistrement du débit des solutions de nettoyage, de désinfection, de rinçage et de séchage passant au travers de chacun des canaux des endoscopes au cours des opérations destinées à assurer leur nettoyage et leur désinfection.

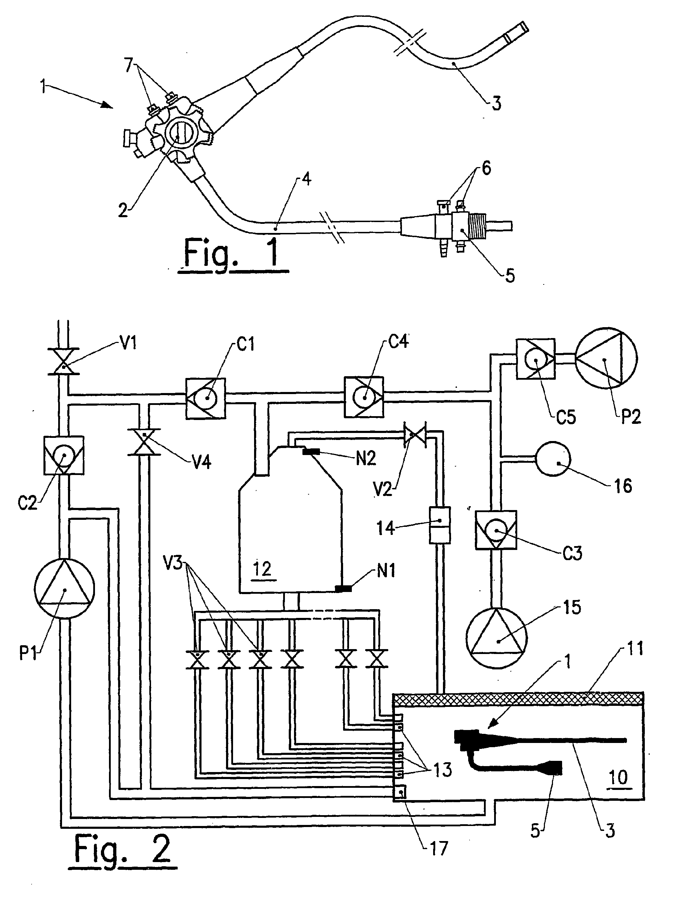

Les endoscopes 1 (figure 1) sont des équipements utilisés dans le milieu hospitalier. Ils comportent un boîtier de manoeuvre 2 montée à l'une des extrémités d'un tube d'exploration 3 destiné à être introduit par un conduit naturel dans une cavité interne du corps d'un patient pour effectuer le diagnostic de lésions ou certains traitements tels que par exemple, l'extraction de corps étrangers, la destruction de tumeurs par coagulation ou résection, l'introduction de médicaments ou de substances opaques aux rayons X. La tête d'exploration est reliée par un second tube 4 à une partie proximale 5 comportant une série de raccords d'injection 6.

Il s'agit d'appareils complexes comportant dans un même tube des fibres optiques guidant la lumière en provenance d'un générateur, des fibres optiques porteuses d'images ou un capteur vidéo (CCD) ainsi que divers canaux tels que canaux opérateurs, canal d'aspiration, canal d'insufflation d'air, canal d'insufflation d'eau, canal érecteur, canal de lavage. Pour des raisons de compacité de l'endoscope, certains d'entre eux peuvent se rejoindre dans leur partie avale pour ne former qu'un seul canal. Cela peut par exemple être le cas des canaux air et eau qui forment un canal commun sur les derniers centimètres du tube d'insertion de l'endoscope et des canaux opérateurs qui rejoignent le canal d'aspiration. Les flux circulant dans l'endoscope peuvent également se mélanger dans une chambre (cage à piston) possédant le même nombre d'entrées que de sorties.

La difficulté réelle de nettoyage et de désinfection de ces appareils concerne ces canaux internes dans lesquels la circulation des fluides peut en raison de leur diamètre (0,5 à 4 millimètres) être nulle ou insuffisante. Une pression insuffisante ne permet pas le passage des solutions et une pression trop importante peut endommager les canaux. Par ailleurs, en raison de l'interrelation des canaux il est difficile pour l'opérateur de s'assurer que la circulation des solutions a été suffisante dans chaque portion de canal. Il est dès lors impossible de s'assurer que le procédé de lavage et de désinfection ont été efficaces.

Pour s'assurer de l'efficacité des divers processus de nettoyage et de désinfection présent sur le marché, qu'ils soient manuels ou automatisés, il est nécessaire de réaliser des prélèvements dans chacune des portions des canaux. Pour cela, un personnel spécialisé (hygiéniste ou pharmacien en général) fait circuler au moyen d'une seringue des solutions de prélèvement. Ces solutions sont capables de décrocher les germes restant éventuellement sur les parois de canaux de l'endoscope ainsi que de neutraliser les reliquats éventuels de désinfectant de nature à fausser les résultats du prélèvement.

Cette manipulation est contraignante car elle nécessite d'irriguer chacune des entrées des différents canaux de l'endoscope l'un après l'autre avec tous les risques éventuels de contamination liés à la manipulation. Elle nécessite également de rincer et de désinfecter à nouveau l'endoscope.

La lourdeur de cette manipulation ainsi que du protocole qui lui est associé, la nécessité de recourir à du personnel spécialisé qui est le plus souvent en nombre limité dans l'hôpital font que ces prélèvement ne sont réalisé que trop rarement.

Un système de nettoyage et désinfection automatisé des endoscopes souples a été décrit précédemment dans le brevet français N° FR 2 705 896. Il permet de faire circuler les fluides sur les parties externes de l'endoscope, ainsi qu'indépendamment dans chacun des canaux internes de l'endoscope. Cependant, ce système ne permet pas de s'assurer automatiquement que les fluides de nettoyage / désinfection /rinçage/ séchage ont effectivement circulé dans chaque portion de chaque canal. Si l'endoscope est improprement raccordé à l'automate ou si l'un ou plusieurs canaux sont partiellement ou totalement obstrués, le débit et la concentration des solutions de nettoyage / désinfection / rinçage / séchage à travers l'endoscope ainsi que les temps de contact peuvent être fortement réduits, voire annulés, entraînant une désinfection incomplète de l'instrument.

En terme de sécurité pour le patient, il s'agit actuellement de la faille la plus importante de ce type de machines. C'est la raison pour laquelle il est demandé aux opérateurs de s'assurer préalablement que chacun des canaux n'est pas obturé et de contrôler en fin de cycle que les canaux sont correctement raccordés.

Certains automates ont cependant été équipés de débitmètres ou de capteurs de pression afin de contrôler le flux ou la pression dans chacun des canaux. Ces procédés essaient tous de déterminer le volume circulant dans les canaux dans une unité de temps. Le volume est déduit à partir d'interprétation d'un capteur de mesure (chute de pression, nombre d'impulsions d'une roue).

Ces solutions se confrontent cependant à de nombreux problèmes :
- Elles s'avèrent coûteuses car elles nécessitent autant de capteurs que de portions de canal à contrôler (jusqu'à 8 selon les endoscopes).
- Afin de permettre un contrôle précis, le débit ou la pression délivrés à l'entrée des canaux ne peut pas être optimisé. Il en découle que les temps de contrôle sont longs, pouvant atteindre plusieurs minutes par canal contrôlé ce qui est de nature à augmenter le temps total des cycles.
- Elles ne garantissent pas qu'un volume minimal correspondant à la saturation du canal contrôlé a effectivement circulé dans le canal.
- Le diagnostic de bonne circulation dans un canal ne repose que sur les indications fournies par un seul capteur. La défaillance de ce capteur peut engendrer une erreur grave de diagnostic.
- Elles nécessitent enfin des étalonnages fréquents et en raison de la diversité des types de canaux d'une marque à l'autre les réglages des seuils s'avèrent être des compromis. Ces réglages déclenchent des alarmes non justifiées qui conduisent les utilisateurs à trop souvent désactiver ces sécurités.

Le dispositif faisant l'objet de la présente invention permet de s'assurer à l'aide d'un nombre limité de dispositifs ne reposant pas sur l'interprétation de capteurs, que les fluides circulent dans chacun des canaux internes de l'endoscope avec un débit bien caractérisé et enregistré pendant chacune des phases de nettoyage, désinfection, rinçage et séchage de l'endoscope.

Grâce à un système permettant d'augmenter la pression du flux passant dans les canaux de l'endoscope et d'assurer qu'un volume connu de solution a bien circulé dans lesdits canaux et permettant de contrôler et d'enregistrer le débit dans chaque canal à une pression donnée, l'invention permet de s'assurer que chacun d'entre eux a bien été nettoyé, désinfecté, rincé et séché par la garantie de présence d'un volume suffisant de la solution concernée pour saturer le canal durant un temps bien déterminé. Elle permet également d'utiliser pour chacun des canaux la pression et le débit optimaux de nature à assurer l'effet mécanique optimal dans un temps le plus bref possible. Elle a finalement pour objet de fournir à l'utilisateur un moyen rapide et fiable de prélever en fin de cycle toutes les portions des canaux internes de l'endoscope afin de pouvoir analyser son état éventuel de contamination.

Le dispositif est constitué d'une chambre hermétique d'un volume connu pourvue de capteur de niveau bas et haut permettant de contrôler son remplissage et sa vidange et dont la partie supérieure est raccordée à un compresseur d'air filtré régulé par un capteur de pression, à une électrovanne de liaison permettant de laisser s'évacuer l'air lors des remplissage de ladite chambre, à une pompe d'injection permettant d'injecter un produit favorisant le séchage et à une pompe de cyclage faisant circuler les solutions nettoyantes et désinfectantes contenues dans une cuve pouvant recevoir au moins un endoscope, cette cuve comportant des injecteurs auxquels les canaux de l'endoscope peuvent tous être individuellement reliés, lesdits injecteurs étant raccordés chacun à la partie inférieure de la chambre hermétique par l'intermédiaire d'une tubulure, une électrovanne d'injection étant disposée entre la chambre hermétique et l'injecteur sur chacune de ces tubulures.

On donne ci-après un exemple de mise en oeuvre dont la portée générique n'est en aucun cas limitée par les particularités ou aux particularités spécifiques à l'exemple choisi pour l'illustration.

Dans le dessin joint :
- la figure 1, déjà mentionnée, représente un endoscope de type courant
- et la figure 2 est le schéma synoptique d'un dispositif conforme à l'invention.

Comme dans le brevet cité plus haut, le dispositif de contrôle objet de la présente demande est composé d'une cuve 10 et comportant un capot 11 équipé d'un joint gonflable relié à une source d'air comprimé permettant d'assurer l'étanchéité de l'ensemble une fois le capot fermé.

Il comporte en particulier une pompe de cyclage P1 qui brasse les solutions dans la cuve 10 dans laquelle est positionné l'endoscope 1. Cette pompe alimente au travers de deux clapet de non-retour C1, C2 une chambre hermétique 12 d'un volume connu préférablement au moins égal à une fois le volume maximal du plus volumineux des canaux d'un endoscope.

La cuve 10 est remplie au moyen d'électrovannes V1 et V4 reliées à un réseau d'eau de rinçage. Cette eau est filtrée à 0,2 microns. Les électrovannes sont reliées au circuit hydraulique en provenance de la pompe de cyclage entre les clapets C1 et C2.

Un corps de chauffe permet de chauffer les sortions avant de les ré-injecter dans la cuve au moyen de deux types d'injecteurs 13, 17 permettant, pour les uns, de faire circuler les solutions autour de la partie externe de l'endoscope 1, pour les autres d'irriguer individuellement les canaux internes de l'appareil.

Le fond de la cuve est raccordé à une pompe de vidange permettant de rejeter les solutions dans le réseau des eaux usées.

Les injecteurs non utilisés seront raccordés à des tubes de section interne inférieur à 5 mm avec une extrémité libre.

Le haut de la chambre hermétique 12 est raccordé à une électrovanne de liaison V2 dont l'aval est raccordé à la cuve 10 ou au capot 11 de celle-ci par l'intermédiaire d'un connecteur rapide 14 aisément accessible de l'extérieur de la machine par l'utilisateur et permettant rapidement et aisément d'injecter dans la chambre au moyen d'une seringue ou d'une pompe une solution, par exemple de prélèvement, qui puisse être recueillie après ouverture d'une ou de plusieurs électrovannes, à l'extrémité d'un ou plusieurs canaux de l'endoscope 1. Le bas de la chambre est raccordée aux électrovannes d'injection V3 qui chacune sont raccordées à un connecteur d'injecteur 13 spécifique dans la cuve lequel connecteur est lui même raccordé à une des entrées de canaux de l'endoscope 1. Une pompe d'injection P2 de liquide de séchage (alcool par exemple) est raccordée au travers d'un clapet de non retour C5 à la ligne en provenance du compresseur d'air entre les clapets C3 et C4.

L'électrovanne de liaison (V2) est raccordé à la cuve (10) ou au capot (11) de la cuve de façon à ce que l'air ainsi que les solutions de remplissage de la chambre hermétique (12) puissent revenir dans la cuve soit au dessus soit au dessous du niveau du liquide contenu dans ladite cuve.

La chambre hermétique 12 est également équipée d'un capteur de niveau bas N1 et d'un capteur de niveau haut N2.

Plusieurs chambres hermétiques de volumes différents peuvent être utilisées conjointement pour contrôler le débit de plusieurs endoscopes ou pour contrôler plus rapidement le débit dans des ensembles de canaux d'un même endoscope ayant des diamètres similaires.

Le système est équipé d'un compresseur 15 d'air filtré qui alimente la chambre hermétique 12 au travers d'une ligne comportant une soupape tarée à une pression donnée ou deux clapets de non retour C3, C4 entre lesquels est intercalé en dérivation un capteur de pression 16 qui permet de réguler la pression délivrée dans la chambre à des valeurs de consigne différentes.

Les produits de nettoyage et de désinfection sont injectés sous forme concentrée dans la cuve 10 au moyen d'un dispositif de dosage. Ils y sont ensuite dilués par brassage à l'aide de la pompe de cyclage P1. Le cycle standard est généralement composé des séquences suivantes :
- Remplissage de la cuve
- Injection du produit de nettoyage
- Activation de la pompe de cyclage, nettoyage de la gaine externe de l'endoscope et irrigation des canaux de l'endoscope
- Vidange de la cuve
- Rinçage de la cuve et des canaux de l'endoscope en eau filtrée
- Remplissage de la cuve
- Injection du produit de désinfection
- Activation de la pompe de cyclage, désinfection de la gaine externe de l'endoscope et des canaux de l'endoscope
- Vidange de la cuve
- Rinçage de la cuve et des canaux de l'endoscope en eau filtrée
- Séchage des canaux

L'ensemble des composants électromécaniques sont pilotés par un automate avec microprocesseur qui en fonction de l'état de capteurs (niveau, pression, température) gère le cycle ainsi qu'un ensemble d'alarme d'interruption. Dans cette cuve peuvent être traités un ou plusieurs endoscopes.

Les endoscopes 1 qui sont traités dans ce dispositif comportent une étiquette ou une puce qui permettent au moyen d'un lecteur de renseigner l'automate sur la marque de l'endoscope, son numéro de série ainsi que son type. L'automate a préalablement été programmé avec les données des fabricants d'endoscope concernant la longueur et le diamètre des canaux qui le constituent.

Pour le raccordement aux entrées de l'endoscope 1, des séparateurs sont préalablement mis en place dans la cage à piston 7 (figure 1) commune aux canaux air et eau de façon à séparer les flux. Ces séparateur sont usinés de façon à maintenir une infime communication entre les deux circuits permettant au désinfectant d'agir et de ne pas créer de zone morte non désinfectable. La mise en communication ne doit pas permettre une fuite supérieure à 30% du flux de chacun d'entre eux.

Pour certains des canaux de l'endoscope, notamment ceux de diamètre interne supérieur à 3 mm, le raccord de connexion à l'endoscope sera équipé d'un dispositif d'obturation qui n'autorise la circulation que si le raccord est correctement connecté sur l'entrée du canal.

Chacune des entrées de l'endoscope est raccordée à un injecteur 13 distinct situé dans la cuve 10 et pouvant être identifié pour le raccordement d'au moins l'un des canaux suivant : canal à biopsie, canal d'aspiration, canal à biopsie auxiliaire ou du canal water-jet, canal air, canal eau, canal érecteur et canal eau auxiliaire.

Nous décrivons ci-dessous le principe du contrôle d'irrigation d'un des canaux avec les solutions en provenance de la cuve à l'aide du dispositif selon l'invention. Ce contrôle s'effectue selon les étapes suivantes :
1) L'état des capteurs de niveau N 1, N2 de la chambre hermétique 12 est contrôlé. Si la chambre n'est pas vide, le compresseur d'air 15 est activé en même temps que les électrovannes de canaux V3 de façon à vider la chambre.
2) Dès que les capteurs de niveau donnent l'indication adéquate, le compresseur 15 est activé jusqu'à ce qu'une valeur de consigne (Pa) enregistrée dans l'automate soit atteinte par le capteur de pression 16.
3) Un temps d'attente (TT) enregistré dans l'automate est observé. Si à la fin de ce temps la pression dans la chambre est toujours supérieure à (Pa-x), la séquence de contrôle est continuée. Dans le cas contraire, une alarme indique qu'une fuite est présente sur le circuit et que le test ne peut être mené à bien.
   Cette opération permet de vérifier l'étanchéité de la chambre hermétique 12 avant tout remplissage, ainsi que de toutes les électrovannes de canaux V3 qui lui sont raccordées.
   Les valeurs (TT),(Pa) et (x) sont déterminées en fonction des sections et des volumes des circuits et de la chambre hermétique 12 de façon à ce que le temps du diagnostic soit optimisé.
4) L'électrovanne de liaison V2 est ouverte et la pompe de cyclage P1 est activée de façon à remplir la chambre hermétique 12 avec la solution en provenance de la cuve 10. Le temps (T1) pour atteindre le niveau haut (capteur N2) est chronométré et enregistré dans l'automate.
5) Dès que le capteur de niveau haut N2 indique que la chambre est pleine l'action est maintenue durant un temps d'attente égal à la moitié du temps de remplissage (T1/2) de façon à s'assurer que le circuit est saturé jusqu'à l'électrovanne de liaison V2.
6) Simultanément :
   - la pompe de cyclage P1 est désactivée,
   - l'électrovanne de liaison V2 est fermée.
   - l'électrovanne V3 raccordée au canal à contrôler est ouverte.
   - le compresseur d'air 15 est activé tant que la pression indiquée par le capteur de pression 16 n'excède pas la pression maximale (PM) autorisée pour l'irrigation du canal connecté à l'électrovanne de canal V3 ouverte.
   - Un chronométrage est effectué jusqu'à ce que le capteur de niveau bas N1 indique que la chambre 12 est vide.

   La valeur de la pression (PM) à appliquer dans la chambre doit permettre d'injecter le volume de solution emprisonné dans la chambre dans le temps le plus court sans cependant endommager les canaux de l'endoscope. Elle est comprise selon les canaux entre 400 et 3500 mbars.
7) La connaissance du volume et du temps permet de déterminer le débit, de l'enregistrer dans une mémoire du microprocesseur et de le comparer à un débit de référence d'un endoscope de même type de ce même canal qui avait préalablement été enregistré dans l'automate en usine (lorsque l'endoscope est connu) ou lors de l'installation de la machine (si l'endoscope est un modèle peut répandu). Le temps de référence obtenu pourra avantageusement être enregistré dans l'automate ou dans une base de données externe connectée à l'automate.
   Si le temps est supérieur à la valeur de référence majorée d'un coefficient (KB), il pourra être conclu que le canal est obstrué.
   Si le temps est inférieur à la valeur de référence minorée d'un coefficient (KD), il pourra être conclu que le canal est déconnecté ou qu'une fuite est présente entre la sortie de l'électrovanne V3 et l'entrée du canal de l'endoscope.
   Dans le cas où les débits sont dans la limite acceptable des valeurs préenregistrée, elles seront imprimés en fin de cycle sur le ticket de validation du cycle ou transféré sur un support de stockage. Dans le cas contraire le cycle sera interrompu et un message indiquera la cause ainsi que le canal à l'origine du défaut.
8) Si l'endoscope 1 est pourvu de faisceau de canaux ayant une portion commune (canal biopsie et aspiration par exemple), la séquence ci-dessus sera réitérée mais en ouvrant en étape 5 l'ensembles des électrovannes raccordées à des canaux ayant des parties communes avec le canal à traiter, par exemple canal à biopsie et canal d'aspiration.
9) La connaissance du volume et du temps permet de déterminer le débit, de l'enregistrer dans une mémoire du microprocesseur et de le comparer à un débit de référence caractéristique de la vidange de la chambre hermétique 12 simultanément au travers de tous les canaux d'une même chambre.
   Dans le cas où les débits sont dans la limite acceptable des valeurs préenregistrée, ils seront imprimés en fin de cycle sur le ticket de validation du cycle ou transféré sur un support de stockage. Dans le cas contraire le cycle sera interrompu et un message indiquera la cause ainsi que le canal à l'origine du défaut.
   Si le temps est inférieur à la valeur de référence minorée d'un coefficient (KDG), il pourra être conclu que l'un des canaux du faisceau est déconnecté ou qu'une fuite est présente entre la sortie de l'électrovanne V3 et l'entrée du canal de l'endoscope.
10) A la fin des phases de nettoyage et de désinfection et durant la vidange de la cuve 10, l'électrovanne de liaison V2 est ouverte ainsi que l'électrovanne de remplissage V1 en d'eau filtrée durant le temps nécessaire à rincer la chambre 12.
11) Puis les électrovannes V1, V2 sont fermées et le compresseur 15 est activé en même temps que les électrovannes de canaux V3 sont ouvertes et ce jusqu'à ce que le capteur de niveau N1 indique que la chambre 12 est vide. Les canaux ont ainsi été rincés avec de l'eau claire.
12) L e compresseur 15 est activé et les électrovannes de canaux V3 sont ouvertes durant le temps nécessaire à l'égouttage de l'endoscope 1.

Cette séquence de contrôle de débit dans les canaux est activée à une ou plusieurs reprises pour l'ensemble des canaux durant chaque phase du cycle avec la solution présente dans la cuve 10 soit :
- durant le remplissage de la cuve en eau
- durant le nettoyage
- durant le rinçage après nettoyage
- durant le second remplissage de la cuve 10
- durant la désinfection
- durant le rinçage post désinfection
- durant le troisième remplissage de la cuve 10
- durant le rinçage

Durant les périodes où le contrôle n'est pas activé, l'ensemble des électrovannes de canaux V3 sont ouvertes de façon à ce que le flux en provenance de l'électrovanne de remplissage V1 (irrigation en eau filtrée) ou de la pompe de cyclage P1 (irrigation en solution nettoyante ou désinfectante) passe librement dans la cuve 10 pour irriguer l'ensemble des canaux.

Le dispositif permet également de contrôler un volume de liquide de séchage (alcool par exemple) injecté dans les canaux. Pour cela, l'électrovanne de liaison V2 ainsi que la pompe P2 de liquide de séchage sont activées jusqu'à ce que le niveau haut (capteur N2) de la chambre 12 soit atteint.

Puis les électrovannes de canaux V3 sont ouvertes ainsi que le compresseur d'air 15 jusqu'à ce que le capteur de niveau bas N1 indique que la cuve 10 est vide, puis durant le temps nécessaire à l'égouttage ou à l'évaporation du liquide de séchage.

En fin de cycle, le capot 11 est ouvert et sans qu'il y ait besoin de déconnecter l'endoscope 1, des prélèvements peuvent être effectués.

Pour cela le raccord rapide 14 sera déconnecté et la ,partie en provenance de la chambre hermétique 12 est raccordée sur l'aval du corps d'une pompe péristaltique. Ce corps (tuyau en silicone) aura préalablement été stérilisé. L'amont du corps de la pompe est raccordé à un flacon de liquide de prélèvement. L'extrémité distale du tube d'exploration 3 de l'endoscope est insérée dans un bocal stérile de prélèvement.

L'opérateur peut alors sélectionner au moyen par exemple des touches de l'écran de saisie de l'automate le canal à prélever. Tant que la touche est activée, la pompe péristaltique et l'électrovanne V3 correspondant au canal sélectionné sont activées. Le liquide de prélèvement poussé par la pompe péristaltique ressort par l'extrémité du canal et est récupéré dans le bocal. Une fois que la quantité suffisante à été récupérée, la touche est désactivée et le prélèvement du canal suivant peut être effectué.

Une séquence peut également être programmée qui automatiquement prélève un canal après l'autre.

L'extrémité de l'endoscope est ensuite retirée du bocal de recueil, le raccord rapide 14 raccordé à la machine et un cycle est relancé de façon à rincer l'endoscope et le désinfecter à nouveau.

Les particularités apparaissant dans la description qui précède donnent à l'objet de l'invention un maximum d'effets utiles qui n'avaient pas été, à ce jour, obtenus par des dispositifs ou procédés similaires.

## Revendications

1. Dispositif de mesure et de contrôle de la circulation des fluides dans les canaux et sur les parties externes d'un endoscope, ayant en particulier pour objet la vérification et l'enregistrement du débit des solutions de nettoyage, de désinfection, de rinçage et de séchage passant au travers de chacun des canaux des endoscopes au cours des opérations destinées à assurer leur nettoyage et leur désinfection,
**caractérisé par** la combinaison, d'une part, d'une cuve (10) pouvant recevoir au moins un endoscope (1), pourvue d'un capot à fermeture étanche et associée à une pompe de cyclage (P1) faisant circuler des solutions de nettoyage et de désinfection de l'extérieur de l'endoscope, ladite cuve étant raccordée à une arrivée d'eau ainsi qu'à une pompe de vidange et comportant des injecteurs (13', 13) internes permettant respectivement de faire circuler les solutions autour des parties externes de l'endoscope (1) et d'irriguer individuellement les canaux internes de l'appareil et, d'autre part, d'un système permettant de faire circuler des fluides appropriés dans chaque canal interne de l'endoscope (1) durant chacune des phases de nettoyage, désinfection, rinçage et séchage, grâce à au moins une chambre hermétique (12) raccordée à sa partie inférieure aux injecteurs (13) de la cuve (10) par l'intermédiaire d'une tubulure et d'électrovannes (V3), un compresseur d'air (15) permettant d'augmenter la pression du flux passant dans lesdits canaux, l'ensemble étant agencé pour permettre d'assurer qu'un volume connu de solution a bien circulé dans lesdits canaux, le système permettant en outre de contrôler et d'enregistrer le débit dans chaque canal à une pression donnée.

2. Dispositif selon la revendication 1 **caractérisé par le fait que** le haut de la chambre hermétique (12) est raccordé à la cuve (10) ou au capot (11) de celle-ci par une conduite pourvue d'une électrovanne de liaison (V2) agencée de manière à ce que l'air ainsi que les solutions de remplissage de la chambre hermétique (12) puissent revenir dans la cuve (10) soit au dessus soit au dessous du niveau du liquide contenu dans ladite cuve.

3. Dispositif selon la revendication 2 **caractérisé par le fait que** la conduite reliant la chambre hermétique (12) à la cuve (10) comporte, à l'aval de l'électrovanne de liaison (V2), un connecteur rapide (14) aisément accessible de l'extérieur de la machine par l'utilisateur et permettant rapidement et aisément d'injecter dans la chambre hermétique (12), au moyen d'une seringue ou d'une pompe, une solution, par exemple de prélèvement, qui puisse être recueillie après ouverture d'une ou de plusieurs électrovannes (V3), à l'extrémité d'un ou plusieurs canaux de l'endoscope 1.

4. Dispositif selon l'une quelconque des revendications précédentes, **caractérisé par le fait qu'**il est équipé d'un corps de chauffe permettant de chauffer les solutions avant de les ré-injecter au moyen des injecteurs (13', 13) dans la cuve (10) ou les canaux de l'endoscope (1).

5. Dispositif selon l'une quelconque des revendications précédentes, **caractérisé par le fait que** la cuve (10) est alimentée par l'intermédiaire de deux électrovannes (V1, V4) reliées d'une part à un réseau d'eau filtrée à 0,2 microns et, d'autre part, au circuit hydraulique en provenance de la pompe de cyclage (P1).

6. Dispositif selon l'une quelconque des revendications précédentes, **caractérisé par le fait que** la chambre hermétique (12) peut être alimentée par la pompe de cyclage (P1) grâce à deux clapets de non-retour (C1, C2).

7. Dispositif selon l'une quelconque des revendications précédentes, **caractérisé par le fait que** certains raccords de connexion entre les injecteurs (13) et l'endoscope (1) sont équipés d'un dispositif d'obturation qui n'autorise la circulation de fluide que si le raccord est correctement connecté sur l'entrée du canal correspondant.

8. Dispositif selon l'une quelconque des revendications précédentes, **caractérisé par le fait qu'**il comporte une pompe d'injection (P2) de liquide de séchage raccordée au travers d'un clapet de non retour (C5) à la tubulure provenant du compresseur d'air (15) entre deux clapets C3 et C4.

9. Dispositif selon l'une quelconque des revendications précédentes, **caractérisé par le fait que** la chambre hermétique (12) est équipée d'un capteur de niveau bas (N1) et d'un capteur de niveau haut (N2) permettant de contrôler son remplissage et sa vidange.

10. Dispositif selon l'une quelconque des revendications précédentes, **caractérisé par le fait qu'**il est équipé d'un lecteur apte à décoder une étiquette ou une puce disposée sur les endoscopes (1) et sur laquelle sont enregistrés sa marque, son numéro de série ainsi que son type.

11. Méthode de mesure et de contrôle de la circulation des fluides dans les canaux et sur les parties externes d'un endoscope, utilisant le dispositif selon les revendications 1 à 10,
**caractérisée en ce que** l'endoscope (1) est disposé dans la cuve (10) d'un appareil de nettoyage et de désinfection piloté par un automate programmable et dont chacune des entrées des canaux est connectée par l'intermédiaire d'injecteurs (13) de ladite cuve et d'électrovannes raccordées par une tubulure au bas d'une chambre hermétique (12) d'un volume connu équipée de deux capteurs de niveau (N1, N2) permettant de contrôler son remplissage et sa vidange, dont la partie supérieure est raccordée à un compresseur (15) d'air filtré régulée par un capteur de pression (16) et à une électrovanne de liaison (V2) permettant de laisser s'évacuer l'air lors des remplissages de ladite chambre, et **en ce que** le temps, pour passer du niveau haut au niveau bas lorsque de l'air est pulsé dans la chambre hermétique (12) à une pression donnée et qu'une ou plusieurs des électrovannes (V3) raccordée à un canal est (sont) ouverte(s), est mesuré et enregistré.

12. Méthode selon la revendication 11, **caractérisée par le fait que** le cycle de nettoyage et de désinfection est composé des séquences suivantes :
- Remplissage de la cuve (10).
- Injection du produit de nettoyage.
- Activation de la pompe de cyclage (P1), nettoyage de la gaine externe de l'endoscope (1) et irrigation des canaux de l'endoscope.
- Vidange de la cuve.
- Rinçage de la cuve et des canaux de l'endoscope en eau filtrée.
- Remplissage de la cuve.
- Injection du produit de désinfection.
- Activation de la pompe de cyclage (P1), désinfection de la gaine externe de l'endoscope et des canaux de l'endoscope.
- Vidange de la cuve.
- Rinçage de la cuve et des canaux de l'endoscope en eau filtrée.
- Séchage des canaux.

13. Méthode selon l'une quelconque des revendications 11 et 12, **caractérisée par le fait que** les étapes du contrôles comprennent successivement :
- Vidange de la chambre hermétique (12) jusqu'au niveau bas.
- Contrôle d'étanchéité à l'air de ladite chambre ainsi que de chacune des électrovannes de canaux (V3) et clapets qui lui sont raccordés.
- Remplissage de la chambre jusqu'au niveau haut (N2) et contrôle du temps de remplissage.
- Vidange de la chambre hermétique et le chronométrage du temps pour atteindre le niveau bas.

14. Méthode selon l'une quelconque des revendications 11 à 13, **caractérisée par le fait que** le contrôle est réitéré sur le même canal de l'endoscope (1) à plusieurs reprises et enregistré, la moyenne des mesures étant comparée à un temps de référence.

## Claims

1. Device for measuring and monitoring the circulation of the fluids in channels and on the external parts of an endoscope, with the particular aim of checking and recording the flow of cleaning, disinfecting, rinsing and drying solutions which pass through each channel of endoscopes during their cleaning and disinfecting,
**characterized by** combining, on the one hand, a vessel (10) capable of receiving at least one endoscope (1), equipped with a sealing cover and associated with a pump (P1) circulating solutions for cleaning and disinfecting the outside of the endoscope, the aforementioned vessel being connected to a water intake together with a draining pump and equipped with internal injectors (13', 13) respectively used to circulate solutions around the external parts of endoscope (1) and irrigate the internal channels of the apparatus individually and, on the other, a system circulating appropriate fluids in each internal channel of endoscope (1) during each cleaning, disinfecting, rinsing and drying phase, thanks to at least one chamber (12) with hermetic connection on its lower part to injectors (13) of vessel (10) through a nozzle and solenoid valves (V3), an air compressor (15) allowing the pressure of the flux passing through the aforementioned channels to be increased, the assembly being arranged to allow a known volume of solution to circulate in the aforementioned channels, the system moreover allowing the control and recording of the flow in each channel at a given pressure.

2. Device according to claim 1 **characterized in that that** the top of hermetic chamber (12) is connected to vessel (10) or to cover (11) by a duct equipped with a solenoid connection valve (V2) arranged so that the air as well as the solutions for filling hermetic chamber (12) can return to vessel (10) either above or below the level of the liquid contained in the said vessel.

3. Device according to claim 2 **characterized in that** the duct connecting hermetic chamber (12) to vessel (10) includes, downstream of connecting solenoid valve (V2), a quick connector (14) which is easily accessible to the user from the outside of the machine and allows quick and easy injection of a solution, for example for sampling, into the hermetic chamber (12) by means of a syringe or a pump, which can then be collected after opening one or several solenoid valves (V3) at the end of one or more channels of endoscope 1.

4. Device according to any of the aforesaid claims, **characterized in that** it is equipped with a heater enabling the heating of the solutions before reinjecting them into vessel (10) by means of injectors (13', 13) or channels of endoscope (1).

5. Device according to any of the aforesaid claims, **characterized in that** vessel (10) is supplied via two solenoid valves (V1, V4) connected on the one hand to a 0.2-micron filtered water supply and on the other to the hydraulic circuit from the circulating pump (P1).

6. Device according to any of the aforesaid claims, **characterized in that** hermetic chamber (12) can be fed by circulating pump (P1) thanks to two non-return valves (C1, C2).

7. Device according to any of the aforesaid claims, **characterized in that** certain connections between injectors (13) and endoscope (1) are equipped with a plugging device which authorizes the circulation of fluid only if the connection is connected correctly onto the intake to the corresponding channel.

8. Device according to any of the aforesaid claims, **characterized in that** it includes a pump (P2) for injecting drying liquid connected through a non-return valve (C5) to the nozzle from air compressor (15) between two valves C3 and C4.

9. Device according to any of the aforesaid claims, **characterized in that** hermetic chamber (12) is equipped with a low level detector (N1) and with a high level detector (N2) for controlling its filling and draining.

10. Device according to any of the aforesaid claims, **characterized in that** it is equipped with a reader able to decode a label or a chip installed on endoscopes (1) and on which are recorded its make, its production number and its type.

11. Method of measuring and controlling the circulation of the fluids in the channels and on the external parts of an endoscope using the device according to claims 1 to 10,
**characterized in that** endoscope (1) is arranged in vessel (10) of a cleaning and disinfecting apparatus operated by a programmable logic controller, each channel entry of which is connected via injectors (13) of the aforesaid vessel and solenoid valves connected by a nozzle at the bottom of a hermetic chamber (12) of known volume equipped with two level detectors (N1, N2) for controlling its filling and its draining, the upper part of which is connected to a compressor (15) of filtered air controlled by a pressure sensor (16) and to a connecting solenoid valve (V2) allowing the air to evacuate when filling the aforesaid chamber, and which can be used to measure and record the time required to pass from the high level to the low level when the air is injected into hermetic chamber (12) at a given pressure and one or more solenoid valves (V3) connected to a channel is/are open.

12. Method according to claim 11, **characterized in that** the cleaning and disinfecting cycle consists of the following sequences:
- Filling of vessel (10),
- Injection of the cleaning product.
- Activation of circulating pump (P1), cleaning of the external sheath of endoscope (1) and irrigation of the endoscope channels.
- Draining of the vessel.
- Rinsing of the vessel and the endoscope channels with filtered water.
- Filling of the vessel.
- Injection of the disinfecting product.
- Activation of circulation pump (P1), disinfecting of the external sheath of the endoscope and the endoscope channels.
- Draining of the vessel.
- Rinsing of the vessel and the endoscope channels with filtered water.
- Drying of the channels.

13. Method according to any of claims 11 and 12, **characterized in that** the inspection stages include the following operations in succession:
- Draining of hermetic chamber (12) down to the low level.
- Checking the airtightness of the aforesaid chamber as well as each of the solenoid valves of the channels (V3) and the disks valve connected to it.
- Filling of the chamber up to high level (N2) and checking the filling time.
- Draining of the hermetic chamber and noting the time to reach the low level.

14. Method according to any of claims 11 to 13, **characterized in that** the check is repeated on same endoscope channel (1) several time and is recorded, the average of the measurements being compared to a reference time.

## Patentansprüche

1. Mess- und Prüfeinrichtung zur Kontrolle des Durchflusses von Fluiden in den Kanälen und äußeren Partien eines Endoskops, insbesondere zum Zweck der Überprüfung und Speicherung der Durchsatzmengen der zur Reinigung, Desinfizierung, Spülung und Trocknung verwendeten Flüssigkeiten, die während der Reinigung- und Desinfizierarbeiten durch die Kanäle des Endoskops geleitet werden,
**gekennzeichnet durch** die Kombination einer Wanne (10), in die mindestens ein Endoskop (1) eingebracht werden kann und die mit einem dicht schließenden Deckel versehen und einer Umwälzpumpe (P1) zugeordnet ist, welche die Lösungen zur Reinigung und Desinfektion außen am Endoskop zirkulieren lässt, wobei die genannte Wanne, die an eine Wasserzufuhrleitung und eine Entleerungspumpe angeschlossen ist, innen mit Spritzdüsen (13', 13) versehen ist, mit denen die Lösungen um die äußeren Partien des Endoskops (1) und auch durch die einzeln inneren Kanäle gespült werden können, sowie mit einem System, mit dem geeignete Fluide während der verschiedenen Reinigungs-, Desinfektions-, Spül- und Trocknungsphasen in jeden der inneren Kanäle des Endoskops (1) geleitet werden können, und dies dank mindestens einer dicht schließenden Kammer (12), die im unteren Teil über einen Stutzen und Magnetventile (V3) an die Spritzdüsen (13) der Wanne (10) angeschlossen ist, wobei mit einem Luftkompressor (15) der Druck des **durch** die Kanäle strömenden Fluids erhöht werden kann, und das Ganze so gestaltet ist, dass sichergestellt ist, dass ein bekanntes Flüssigkeitsvolumen tatsächlich **durch** die genannten Kanäle geströmt ist, und das System außerdem gestattet, den bei einem gegebenen Druck in jedem Kanal fließenden Volumenstrom zu messen und zu speichern.

2. Einrichtung gemäß Anspruch 1, **gekennzeichnet dadurch, dass** die dicht schließende Kammer (12) im oberen Teil mit der Wanne (10) oder ihrem Deckel (11) über eine Leitung mit einem Verbindungs-Magnetventil (V2) verbunden ist, das so angeordnet ist, dass die Luft und die zum Füllen der dicht schließenden Kammer (12) dienenden Lösungen entweder ober- oder unterhalb des Flüssigkeitsstandes der genannten Wanne (10) in diese zurückfließen können.

3. Einrichtung gemäß Anspruch 2, **gekennzeichnet dadurch, dass** die Leitung, welche die dicht schließende Kammer (12) mit der Wanne (10) verbindet, dem Verbindungs-Magnetventil (V2) nachgeschaltet eine für den Bediener leicht von außen zugängliche Schnelltrennkupplung (14) besitzt, so dass es schnell und einfach möglich ist, eine Lösung, zum Beispiel zur Probennahme, mit einer Spritze oder einer Pumpe einzuspritzen und sie nach Öffnen eines oder mehrerer Magnetventile (V3) am Ende eines oder mehrerer Kanäle des Endoskops (1) zu entnehmen.

4. Einrichtung gemäss einem der obenstehenden Ansprüche, **gekennzeichnet dadurch, dass** sie mit einem Heizelement versehen ist, um die Lösungen vor ihrem Wiedereinleiten mit Hilfe der Einspritzdüsen (13', 13) in die Wanne (10) oder die Kanäle des Endoskops zu erwärmen.

5. Einrichtung gemäß einem der vorstehenden Ansprüche, **gekennzeichnet dadurch, dass** die Wanne (10) durch zwei Magnetventile (V1, V4) versorgt wird, die an ein Versorgungsnetz für 0,2 µm-Filterwasser und zusätzlich an die von der Umwälzpumpe (P1) kommende Hydraulikleitung angeschlossen sind.

6. Einrichtung gemäß einem der vorstehenden Ansprüche, **gekennzeichnet dadurch, dass** die dicht schließende Kammer (12) dank der beiden Rückschlagklappen (C1, C2) durch die Umwälzpumpe (P1) versorgt werden kann.

7. Einrichtung gemäß einem der vorstehenden Ansprüche, **gekennzeichnet dadurch, dass** einige Kupplungen zwischen den Spritzdüsen (13) und dem Endoskop (1) mit einem Verschlusssystem ausgerüstet sind, das einen Durchfluss des Fluids nur dann gestattet, wenn die Kupplung am Eingang des entsprechenden Kanals einwandfrei angeschlossen ist.

8. Einrichtung gemäß einem der vorstehenden Ansprüche, **gekennzeichnet dadurch, dass** sie eine Einspritzpumpe (P2) für eine Trocknungsflüssigkeit besitzt, die über eine Rückschlagklappe (C5) zwischen den beiden Klappen C3 und C4 an die vom Luftkompressor (15) kommende Leitung angeschlossen wird.

9. Einrichtung gemäß einem der vorstehenden Ansprüche, **gekennzeichnet dadurch, dass** die dicht schließende Kammer (12) mit einem Niveauschalter für Niedrigstand (N1) und einem Niveauschalter für Höchststand (N2) versehen ist, um ihr Befüllen und Entleeren zu steuern.

10. Einrichtung gemäß einem der vorstehenden Ansprüche, **gekennzeichnet dadurch, dass** sie mit einem Lesegerät versehen ist, das die auf den Endoskopen (1) angebrachten Etiketten oder Mikrochips, auf denen die Marke, die Seriennummer und der Typ des Endoskops angegeben sind, dekodieren kann.

11. Verfahren zur Messung und Prüfung des Durchflusses von Fluiden in den Kanälen und äußeren Partien eines Endoskops unter Einsatz der Einrichtung gemäß Anspruch 1 bis 10,
**gekennzeichnet dadurch, dass** das Endoskop (1) in die Wanne (10) eines von einem programmierbaren Automaten gesteuerten Reinigungs- und Desinfektionsgeräts gebracht wird, dass der Eingang jedes Kanals des Endoskops mit Hilfe von Einspritzdüsen (13) der genannten Wanne und Magnetventilen, die über einen Stutzen im unteren Teil einer dicht schließenden Kammer (12) mit bekanntem Volumen, die mit zwei Niveauschaltern (N1, N2) versehen ist, um das Befüllen und Entleeren zu steuern, angeschlossen sind, und deren oberer Teil an einen, mit Hilfe eines Druckgebers (16) geregelten, gefilterte Druckluft liefernden Luftkompressor (15) und an ein Verbindungs-Magnetventil (V2) angeschlossen ist, das eine Entlüftung der genannten Kammer bei deren Befüllen ermöglicht, und **dadurch**, dass die Zeit gemessen und gespeichert wird, die nötig ist, um vom oberen zum unteren Niveau zu gelangen, wenn der dicht schließenden Kammer (12) bei einem gegebenen Druck Luft entnommen wird, und eines oder mehrere der an einen Kanal angeschlossen Magnetventile (V3) offen sind.

12. Verfahren gemäß Anspruch 11, **gekennzeichnet dadurch, dass** der Reinigungs- und Desinfektionszyklus aus folgenden Sequenzen besteht:
- Befüllen der Wanne (10),
- Einspritzen des Reinigungsmittels,
- Einschalten der Umwälzpumpe (P1), Reinigung des äußeren Schutzrohres des Endoskops (1) und Berieselung der Kanäle des Endoskops,
- Entleeren der Wanne.
- Spülen der Wanne und der Kanäle des Endoskops mit gefiltertem Wasser,
- Befüllen der Wanne,
- Einspritzen des Desinfektionsmittels,
- Einschalten der Umwälzpumpe (P1), Desinfizieren des äußeren Schutzrohres des Endoskops (1) und der Kanäle des Endoskops,
- Entleeren der Wanne,
- Spülen der Wanne und der Kanäle des Endoskops mit gefiltertem Wasser,
- Trocknen der Kanäle.

13. Verfahren gemäß einem der Ansprüche 11 und 12, **gekennzeichnet dadurch, dass** folgende Prüfschritte aufeinanderfolgend ablaufen:
- Entleeren der dicht schließenden Kammer (12) bis zum Erreichen des unteren Niveaus,
- Prüfung der genannten Kammer sowie jedes Magnetventils der Kanäle (V3) und der ihr angeschlossenen Klappen auf Luftdichtigkeit.
- Befüllen der Kammer bis zum oberen Niveau (N2) und Messung der Befüllzeit,
- Entleeren der dicht schließenden Kammer und Messung der Zeit bis zum Erreichen des unteren Niveaus.

14. Verfahren gemäß einem der Ansprüche 11 bis 13, **gekennzeichnet dadurch, dass** die Prüfung mehrmals am gleichen Kanal des Endoskops (1) wiederholt und aufgezeichnet und der Mittelwert der Messungen mit einer Bezugszeit verglichen wird.
